# NEW EUROPEAN PATENT SPECIFICATION

(11) **EP 1 676 562 B2**
(45) Date of publication and mention of the opposition decision: **02.03.2016**
(45) Mention of the grant of the patent: 04.07.2012
(21) Application number: 04029770.7
(22) Date of filing: 16.12.2004
(51) Int. Cl.: A61K 8/36, A61K 8/362, A61K 8/365, A61K 8/34, A61Q 5/02, A61Q 5/12, A61Q 17/04

(54) **Composition for the hair containing uv filters**
Haarzubereitung enthaltend uv Filter
Compositions pour les cheveux contenant des filtres uv

(43) Date of publication of application: 05.07.2006
(73) Proprietor: Kao Germany GmbH, 64297 Darmstadt (DE)
(72) Inventor: Molenda, Michael, 60598 Frankfurt (DE); Lateulere, Magali, 64297 Darmstadt (DE); Golinski, Frank Dr., 64372 Ober-Ramstadt (DE)

(56) References cited:
- EP-A- 0 770 399
- EP-A- 1 174 112
- EP-A1- 1 656 967
- EP-A2- 1 568 350
- WO-A-03/013456
- WO-A1-95/20375
- DE-A1- 10 219 433
- FR-A1- 2 795 319
- US-A1- 2002 009 423
- OTTE I., DR. ET AL: 'Wörterbuch der Kosmetik', vol. 4, 1997, WISSENSCHAFTLICHE VERLAGSGESELLSCHAFT MBH, STUTTGART page 204
- Datasheet Uvinul MS40, April 1999

## Description

The present invention is related to a conditioning composition for hair comprising UV absorbing substances. The conditioning composition of the present invention can be in the form of a shampoo, cleansing - conditioning composition, or in the form of a conditioner used after washing hair with cleansing compositions.

The use of UV absorbers in hair conditioning compositions is known in the prior art. The purpose is obviously protection hair from undesirable damaging effects of UV light. The prerequisite of an optimum protection is the deposition of the UV filters onto hair surface. In practice there are difficulties observed achieving this especially when water-soluble UV absorbing substances are used.

In order to overcome such problems, leave in type of products have become popular. As those products are not rinsed off from hair, high deposition rates can be achieved.

For products which are rinsed off from hair after application and certain processing time such as shampoo and rinse off conditioners, the deposition rates have been evaluated to be very low and subsequently lower effects have been observed.

The objective of the current invention is to find out a composition from which water soluble anionic UV filters as formulated show high deposition onto hair. It should be noted that the present invention is mainly concerned rinse off formulations, however, the application of the compositions of the current invention as leave in product is not excluded.

It has surprisingly been found out that deposition of water soluble anionic UV filters is enhanced very much from a composition comprising at the same time at least one hair conditioning agent, at least one organic solvent and having pH lower than 4.5. The compositions show optimum performance in hair protecting and excellently superior performance in hair shine improving, making hair excellently manageable and softness of hair. The compositions of the present invention improve combability or, in other words prevent loss of combability as a result of the damaging effects of sun and/or UV light, volume and body of hair. After using the compositions of present invention, hair feels nicer and more natural when touching. The effects mentioned are more pronounced on repeated usage.

EP 1174112 discloses hair cosmetic compositions comprising organic acid, organic solvent, cationic surfactant and higher alcohol and having pH in the range of 2 to 6 for improving hair shine. Additionally, WO 2004/047777 discloses leave-in compositions for hair comprising malic and lactic acids and organic solvents for improving shine, setting and touch feeling. Both documents are silent on improving deposition of UV filters onto hair.

The UV filters are those water soluble ones for the purpose of protecting hair. In other words, anionic UV filters are suitably used in the compositions of the present invention. Suitable UV-absorbing substances is are: 4-Aminobenzoic acid and the esters and salts thereof, 2-phenyl benzimidazole-5-sulfonic acid and the alkali and amine salts thereof, 4-dimethyl aminobenzoic acid and the esters and salts thereof, cinnamic acid and the esters and salts thereof, 4-methoxycinnamic acid and the esters and salts thereof, salicylic acid and the esters and salts thereof, 2.4-dihydroxybenzophenone, 2.2'.4.4'-tetrahydroxybenzophenone, 2-hydroxy-4-methoxybenzophenone and its 5-sulfonic acid or the sodium salt thereof, 2.2'-dihydroxy-4.4'-dimethoxybenzophenone, 2-hydroxy-5-chlorobenzophenone, 2.2'-dihydroxy-4-methoxybenzophenone, 2.2'-dihydroxy-4.4'-dimethoxy-5.5'-disulfobenzo-phenone or the sodium salt thereof, 2-hydroxy-4-octyloxybenzophenone, 2-hydroxy-4-methoxy-4'-methylbenzophenone, 3-benzyl-idenecampher, 3-(4'-sulfo)-benzyl-idenebornane-2-one and the salts thereof and/or 3-(4'-methyl benzylidene)-DL-campher. Among those water soluble anionic benzophenones such as benzophenone-4 are especially preferred The amount of the UV-absorber ranges typically from about 0.01 % to 5%, more preferably from 0.01 % to 4 %, most preferably 0.05 to 2.5% by weight, calculated to the total composition.

The pH of the compositions according to the present invention is below 4.5 and preferably in the range of 2.0 to 4.0, more preferably 2.5 to 3.8., for achieving enhanced and optimum deposition of UV filters of especially anionic water soluble.

In principal pH of the compositions can be adjusted with any organic and/or inorganic acids or their mixture. Some of them to mention are phosphoric acid, hydrochloric acid as the inorganic ones and to the organic acids the well known citric acid. However, the best conditioning effect is observed when the carboxylic acids and especially those of with hydroxycarboxylic acids and/or dicarboxylic acids are included into the composition for adjusting pH. In those cases where selected hydroxycarboxylic acid and/or dicarboxylic acid concentration is not enough to reach the selected pH, other organic and inorganic acids can as well be used to adjust pH to the required value. The hydroxycarboxylic acids useful in the compositions of the present invention are lactic acid, glycolic acid, hydroxyacrylic acid, glyceric acid, malic acid and tartaric acid and of the dicarboxylic acids are malonic acid, succinic acid, glutaric acid, adipic acid, maleic acid, fumaric acid and phtalic acid.

Compositions according to invention comprise at least one hydroxycarboxilic acid and/or dicarboxylic acid. Combinations of two or more hydroxycarboxylic acids and/or dicarboxylic acids are also within the scope of the invention. It should be noted that hydroxycarboxylic acid and dicarboxylic acid comprising compositions are also within the scope of the present invention. Especially preferred hydroxycarboxylic acids are the lactic and malic acids. Malic acid is also a dicarboxy acid. The hydroxycarboxylic acid and/or dicarboxylic acid is the malic acid.

The concentration of the acidic components in the compositions of the present invention is in the range from 0.5 to 5% by weight, preferably 0.25 to 4% by weight and more preferably 0.5 to 3.5% by weight calculated to the total composition. In the preferred form of carrying out the invention these values as well correspond to the total hydroxycarboxylic acid and/or dicarboxylic acid content of the compositions of the present invention. In another preferred embodiment of the invention, the compositions of the present invention comprise malic acid at a concentration of 0.5% by weight calculated to the total composition.

Conditioning composition comprises organic solvents such as ethanol, propanol, isopropanol, benzyl alcohol, benzyloxyethanol, ethoxydiglycol, alkylene carbonates such as ethylene carbonate and propylene carbonate, phenoxyethanol, butanol, isobutanol, cyclohexane, cyclohexanol, hexyleneglycol, ethylenecarbonate, propyleneglycol, polypropyleneglycols, ethyleneglycol monoethylether, ethylene glycol monobutyl ether, ethylene glycol monophenyl ether, 1-phenylethylalcohol, 2-phenylethylalcohol, o-methoxyphenol. The most preferred ones are benzyl alcohol, ethanol, benzyloxyethanol propylene glycol and polypropylene glycol. Concentration of organic solvents should not exceed 10% by weight, preferably in the range of 0.1 to 7.5%, more preferably 0.1 to 5% by weight calculated to total composition in compositions designed for cleansing and conditioner / treatment in emulsion form.

The compositions of the present invention can be either a conditioning -cleansing composition or a conditioning composition typically used after use of cleansing compositions.

The composition of the present invention comprises hair-conditioning agents in any type of composition mentioned above. Conditioning agents can be selected from oily substances, non-ionic substances, cationic amphiphilic ingredients, cationic polymers or their mixtures.

Oily substances are selected from such as silicone oils, either volatile or nonvolatile, natural and synthetic oils. Among silicone oils those can be added to the compositions include dimethicone, dimethiconol, polydimethylsiloxane, DC fluid ranges from Dow Corning, natural oils such as olive oil, almond oil, avocado oil, wheatgerm oil, ricinus oil and the synthetic oils, such as mineral oil, isopropyl myristate, palmitate, stearate and isostearate, oleyl oleate, isocetyl stearate, hexyl laurate, dibutyl adipate, dioctyl adipate, myristyl myristate and oleyl erucate.

Non-ionic conditioning agents may be polyols such as glycerin, glycol and derivatives, polyethyleneglycoles known with trade names Carbowax PEG from Union Carbide and Polyox WSR range from Amerchol, polyglycerin, polyethyleneglycol mono or di fatty acid esters having general formula

R₁ CO (O CH₂ CH₂)ₙ OH

or

R₁ CO (O CH₂ CH₂)ₙ O OC R₂

where R₁ and R₂ are independent from each other saturated, unsaturated or branched or non-branched alkyl chain with 7 to 21 C atoms and n is typically 2 - 100.

In one of the preferred from of the present invention, conditioning compositions comprise at least one cationic polymer as conditioning agent. Suitable cationic polymers are those of best known with their CTFA category name Polyquaternium. Typical examples of those Polyquaternium 6, Polyquaternium 7, Polyquaternium 10, Polyquaternium 11, Polyquaternium 16, Polyquaternium 22 and Polyquaternium 28.

As well those polymers known with their CTFA category name Quaternium are suitable. Those are for example Quaternium-8, Quaternium-14, Quaternium-15, Quaternium-18, Quaternium-22, Quaternium-24, Quaternium-26, Quaternium-27, Quaternium-30, Quaternium-33, Quaternium-53, Quaternium-60, Quaternium-61, Quaternium-72, Quaternium-78, Quaternium-80, Quaternium-81, Quaternium-81, Quaternium-82, Quaternium-83 and Quaternium-84.

It has further been found out that especially those of cationic cellulose type polymers known as Polymer JR type from Amerchol such as Polyquaternium 10 or cationic guar gum known with trade name Jaguar from Rhône-Poulenc and chemically for example Guar hydroxypropyl trimonium chloride, are preferred ones. Furthermore, chitosan and chitin can also be included in the compositions as cationic natural polymers. In this context reference is also made to the cationic polymers disclosed in DE 25 21 960, 28 11 010, 30 44 738 and 32 17 059, as well as to the products described in EP-A 337 354 on pages 3 to 7. It is also possible to use mixtures of various cationic polymers.

The most preferred cationic polymers are those of cationic cellulose derivatives, cationic guar gum derivatives, polyquaternium 6 and polyquaternium 7.

The cationic polymers also include the quaternized products of graft polymers from organopolysiloxanes and polyethyl oxazolines described in EP-A 524 612 and EP-A 640 643.

Conditioning compositions of the present invention can comprise additionally one or more cationic surfactant(s) as conditioner presented with the general formula 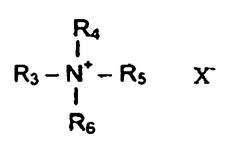 where R₃ is a saturated or unsaturated, branched or non-branched alkyl chain with 8-22 C atoms or

R₇ CO NH (CH₂)ₙ

where R₇ is saturated or unsaturated, branched or non-branched alkyl chain with 7-21 C atoms and n has value of 1 ― 4,
or

R₈ CO O (CH₂)ₙ

where R₈ is saturated or unsaturated, branched or non-branched alkyl chain with 7-21 C atoms and n has value of 1 - 4, and
R₄ is hydrogen or unsaturated or saturated, branched or non-branched alkyl chain with 1 - 4 C atoms or

R₇ CO NH (CH₂)ₙ

or

R₈CO O (CH₂)ₙ

where R₇, R₈ and n are same as above.

R₅ and R₆ are hydrogen or lower alkyl chain with 1 to 4 carbon atoms, and X is anion such as chloride, bromide, methosulfate.

Typical examples of those ingredients are cetyl trimethyl ammonium chloride, stear trimonium chloride, behentrimonium chloride, stearamidopropyl trimonuim chloride, dioleoylethyl dimethyl ammonium methosulfate, dioleoylethyl hydroxyethylmonium methosulfate.

Amido amines may as well be used as a conditioning cationic surfactant in the compositions of the present invention. Typical non-limiting example is stearamidopropylamine known with a trade name Tego Amid S18 from Degussa.

The compositions according to the invention may also comprise further conditioning substances such as protein hydrolyzates and polypeptides, e.g., keratin hydrolyzates, collagen hydrolyzates of the type "Nutrilan^{R}" or elastin hydrolyzates, as well as also in particular plant protein hydrolyzates, optionally, cationized protein hydrolyzates, e.g., "Gluadin^{R}".

Typical concentration range for any of those conditioners of cationic polymers, silicon oil and derivatives and cationic surfactants can be 0.01 - 10% by weight, preferably 0.01 - 7.5% by weight, more preferably 0.05 - 5% and most preferably 0.1 - 3% by weight calculated to the total composition. It should be noted that especially non-cleansing conditioning type of the products contain higher concentrations of the cationic surfactants which are at the same time if desired can be emulsifying agent. In cleansing and conditioning type of preparations, concentration of cationic surfactants is relatively lower.

Conditioning compositions of the present invention can be a cleansing composition (cleansing-conditioning composition). Cleansing conditioning compositions of the present invention comprise at least one surfactant selected from anionic, non-ionic and/or amphoteric or zwitterionic surfactants at a concentration range of 1 to 50%, preferably 5 to 40% and more preferably 5 to 30%, and most preferably 5 to 25% by weight, calculated to the total composition.

In an embodiment of the present invention cleansing conditioning composition of the present invention, comprises at least one anionic, at least one nonionic surfactant. More preferably the compositions further comprise additionally at least one amphoteric surfactant.

Anionic surfactants suitable within the scope of the invention are preferably present in an amount from 1 to about 30%, preferably 2 to 20% and most preferably 2 - 15%, by weight, calculated to the total composition.

These are anionic surfactants of the sulfate, sulfonate, carboxylate and alkyl phosphate type, especially, of course, those customarily used in shampoo compositions, for example, the known C₁₀-C₁₈- alkyl sulfates, and in particular the respective ether sulfates, for example, C₁₂-C₁₄-alkyl ether sulfate, lauryl ether sulfate, especially with 1 to 4 ethylene oxide groups in the molecule, monoglyceride (ether) sulfates, fatty acid amide sulfates obtained by ethoxylation and subsequent sulfatation of fatty acid alkanolamides, and the alkali salts thereof, as well as the salts of long-chain mono- and dialkyl phosphates constituting mild, skin-compatible detergents.

Additional anionic surfactants useful within the scope of the invention are α-olefin sulfonates or the salts thereof, and in particular alkali salts of sulfosuccinic acid semiesters, for example, the disodium salt of monooctyl sulfosuccinate and alkali salts of long-chain monoalkyl ethoxysulfosuccinates.

Suitable surfactants of the carboxylate type are alkyl polyether carboxylic acids and the salts thereof of the formula

R₉- (C₂H₄O)ₙ- O - CH₂COOX,

wherein R₉ is a C₈-C₂₀-alkyl group, preferably a C₁₂-C₁₄-alkyl group, n is a number from 1 to 20, preferably 2 to 17, and X is H or preferably a cation of the group sodium, potassium, magnesium and ammonium, which can optionally be hydroxyalkyl-substituted, as well as alkyl amido polyether carboxylic acids of the general formula 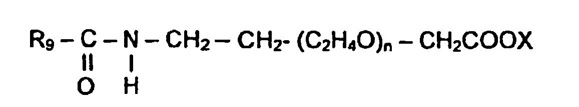 wherein R₉ and X have the above meanings, and n is in particular a number from 1 to 10, preferably 2.5 to 5.

Such products have been known for some time and are on the market, for example, under the trade name "AKYPO® " and "AKYPO-SOFT® ".

Also useful are C₈-C₂₀-acyl isethionates, alone or in admixture with other anionic surfactants, as well as sulfofatty acids and the esters thereof.
It is also possible to use mixtures of several anionic surfactants, for example an ether sulfate and a polyether carboxylic acid or alkyl amidoether carboxylic acid.

An overview of the anionic surfactants used in liquid body cleansing compositions can furthermore be found in the monography of K. Schrader, "Grundlagen und Rezepturen der Kosmetika", 2nd Ed.(1989, Hüthig Buchverlag), pp. 595-600 and pp. 683 to 691.

Further suitable anionic surfactants are also C₈-C₂₂-acyl aminocarboxylic acids or the water-soluble salts thereof. Especially preferred is N-lauroyl glutamate, in particular as sodium salt, as well as, for example, N-lauroyl sarcosinate, N-C₁₂₋C₁₈-acyl asparaginic acid, N-myristoyl sarcosinate, N-oleoyl sarcosinate, N-lauroyl methylalanine, N-lauroyl lysine and N-lauroyl aminopropyl glycine, preferably in form of the water-soluble alkali or ammonium, in particular the sodium salts thereof, preferably in admixture with the above-named anionic surfactants.

Further surfactants in the conditioning cleansing compositions according to the invention are nonionic surfactants in admixture with anionic surfactants.

These are described in Schrader, I.c., on pages 600-601 and pp. 694-695. Especially suited are alkyl polyglucosides of the general formula

R₁₀-O-(R₁₁O)ₙ-Zₓ,

wherein R₁₀ is an alkyl group with 8 to 18 carbon atoms, R₁₁ is an ethylene or propylene group, Z is a saccharide group with 5 to 6 carbon atoms, n is a number from 0 to 10 and x is a number between 1 and 5.

These alkyl polyglucosides have recently become known in particular as excellent skin-compatible, foam improving agents in liquid detergents and body cleansing compositions, and are present in an amount from about 1 % to 15 %, in particular from 1 % to 10 % by weight, calculated to the total composition.

Mixtures of anionic surfactants and alkyl polyglucosides as well as the use thereof in conditioning cleansing compositions are already known, for example, from EP-A 70 074. The alkyl polyglucosides disclosed therein are basically also suited within the scope of the present invention; as well as the mixtures of sulfosuccinates and alkyl polyglucosides disclosed in EP-A 358 216.

Further nonionic surfactant components are, for example, long-chain fatty acid mono- and dialkanolamides, such as coco fatty acid monoethanolamide and myristic fatty acid monoethanolamide, which can also be used as foam enhancers, preferably in amounts from about 1 % to about 5 % by weight.

Further additionally useful nonionic surfactants are, for example, the various sorbitan esters, such as polyethylene glycol sorbitan stearic acid ester, fatty acid polyglycol esters or poly-condensates of ethyleneoxide and propyleneoxide, as they are on the market, for example, under the trade name "Pluronics^{R}", as well as fatty alcohol ethoxylates.

Further suitable nonionic surfactants are amineoxides which may be present in an amount from 0.25 % to 5 % by weight, calculated to the total composition. Such amineoxides are state of the art, for example C₁₂-C₁₈- alkyl dimethyl amineoxides such as lauryl dimethyl amineoxide, C₁₂-C₁₈- alkyl amidopropyl or -ethyl amineoxides, C₁₂-C₁₈ -alkyl di(hydroxyethyl) or (hydroxypropyl) amineoxides, or also amineoxides with ethyleneoxide and/or propyleneoxide groups in the alkyl chain. Such amineoxides are on the market, for example, under the trade names "Ammonyx® ", "Aromox® " or "Genaminox® ".

Further nonionic surfactants useful in the compositions according to invention are C₁₀-C₂₂-fatty alcohol ethoxylates at a concentration of 0.5 to 10%, preferably 0.5 to 5% by weight, calculated to total composition. Especially suited are C₁₀-C₂₂₋fatty alcohol ethers, the alkyl polyglycol ethers known by the generic terms "Laureth", "Myristeth", "Oleth", "Ceteth", "Deceth", "Steareth" and "Ceteareth" according to the CTFA nomenclature, including addition of the number of ethylene oxide molecules, e.g., "Laureth-16": The average degree of ethoxylation thereby ranges between about 2.5 and about 25, preferably about 10 and about 20.

As further surfactant component, the compositions according to the invention can also contain amphoteric or zwitterionic surfactants, for example in an amount from about 0.5 % to about 15 %, preferably from about 1 % to about 10 %, by weight, calculated to the total composition. It has especially been found out that addition of zwitterionic or amphoteric surfactants enhances foam feeling in terms of creaminess, foam volume and as well as skin compatibility is improved. For achieving milder formulations anionic surfactant, especially of sulphate types, to amphoteric surfactant ratio should be in the range of 10:1 to 1:1, preferably 5:1 to 1:1.

Useful as such are in particular the various known betaines such as alkyl betaines, fatty acid amidoalkyl betaines and sulfobetaines, for example, lauryl hydroxysulfobetaine; long-chain alkyl amino acids, such as cocoaminoacetate, cocoaminopropionate and sodium cocoamphopropionate and -acetate have also proven suitable.

In detail, it is possible to use betaines of the structure 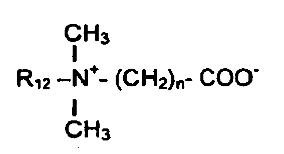 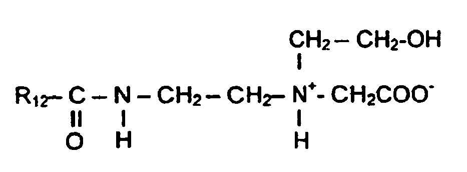 wherein R₁₂ is a C₈-C₁₈-alkyl group and n is 1 to 3;
sulfobetaines of the structure 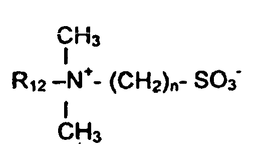 wherein R₁₂ and n are same as above;
and amidoalkyl betaines of the structure 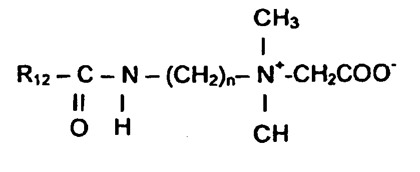 wherein R₁₂ and n are same as above.

Solubilizers may be added to the compositions, in particular cleansing compositions, especially when oily substances are chosen as conditioning agents and fragrance oils with highly lipophilic properties. Typical solubilizers may be hydrogenated castor oil known with the trade mark Cremophor RH series from BASF. It should be noted that as well the surfactant mixture can be a good solubilizer for fragrance oils. Typical concentration of the solubilizers can be in the range of 0.01 - 2% by weight, preferably 0.1 - 1% by weight, calculated to total composition.

Further conditioning additives are hair conditioning and/or styling polymers into either cleansing or conditioning type. These may be nonionic polymers, preferably alcohol- and/or water-soluble vinyl pyrrolidone polymers, such as a vinyl pyrrolidone homopolymers or copolymers, in particular with vinyl acetate. Useful vinyl pyrrolidone polymers are, e.g., those known by the trade name "Luviskol®", for example, the homopolymers "Luviskol® K 30, K 60 and K 90", as well as the water-or alcohol-soluble copolymers from vinyl pyrrolidone and vinyl acetate, distributed by BASF AG under the trade name "Luviskol® VA 55 respectively VA 64". Further possible nonionic polymers are vinyl pyrrolidone/vinyl acetate/vinyl propionate copolymers such as "Luviskol® VAP 343", vinyl pyrrolidone/(meth)acrylic acid ester copolymers, as well as chitosan derivatives.

Amphoteric polymers are found to be useful in conditioning composition of any type of the present invention. They are incorporated alone or in admixture with at least one additional cationic, nonionic or anionic polymer, particularly copolymers of N-octyl acrylamide, (meth)acrylic acid and tert.-butyl aminoethyl methacrylate of the type "Amphomer®"; copolymers from methacryl oylethyl betaine and alkyl - methacrylates of the type "Yukaformer®", e.g., the butyl methacrylate copolymer "Yukaformer® Am75"; copolymers from monomers containing carboxyl groups and sulfonic groups, e.g., (meth)acrylic acid and itaconic acid, with monomers such as mono- or dialkyl amino alkyl(meth)acrylates or mono- or dialkyl - aminoalkyl (meth)acrylamides containing basic groups, in particular amino groups; copolymers from N-octyl acrylamide, methyl methacrylate, hydroxypropyl methacrylate, N-tert.-butyl aminoethyl methacrylate and acrylic acid, as well as the copolymers known from US-A 3,927,199, are applicable.

Conditioning and cleansing composition of the present invention can be transparent as well as pearly. Transparency of the composition is judged by naked eye in a transparent shampoo bottle with a thickness not more than 5 cm. In the case a transparent appearance is wished, the following ingredients are not essential. However, pearl-shiny appearance is achieved with those dispersed in cleansing color-enhancing compositions in crystalline form, i.e. so called pearlshine or pearlizing agents. The preferred once are PEG-3 distearate and ethylene glycol distearate. The concentration of those can typically be from 0.1 to 3%, preferably 0.5 to 2% by weight, calculated to the total composition. These compounds are preferably added to the compositions in admixture with anionic, nonionic and/or amphoteric surfactants. Such kind of mixtures is available commercially.

Hair cleansing conditioning compositions of the present invention can be in the form of conventional liquid thickened shampoo, as well in the form of ready to use foam, delivered either from a pump-foamer or from an aerosol bottle. In the case that an aerosol foam preparation is preferred, propellant gas must be added to the formulation. The suitable propellant gasses are carbondioxide, dimethylether and alkanes such as butane propane or their mixtures.

Conditioning compositions of the present invention can be in the form of emulsions, solutions, gels and dispersions. In the case that solutions and/or gels forms are preferred the appearance can be either with a transparent or opaque. As a product form, foam is as well suited when packed into a pressurized can or delivered through a pump-foamer (non-aerosol). In the case that an aerosol foam preparation is preferred, propellant gas must be added to the formulation. The suitable propellant gasses are carbondioxide, dimethylether and alkanes such as butane, propane, isobutane or their mixtures.

The emulsion type of conditioning compositions of the present invention comprise additionally at least one fatty alcohol of the following formula

R₁₃-OH

where R₁₃ is a saturated or unsaturated, branched or non-branched fatty acyl chain with 8 - 24 C atoms. Concentration of fatty alcohols is usually less than 20%, preferably less than 15% by weight calculated to total composition. Typical examples to the most useful fatty alcohols are myristyl alcohol, palmityl alcohol, cetyl alcohol, stearyl alcohol, behenyl alcohol and their mixtures. As a mixed fatty alcohol the mostly used one is the cetearyl alcohol as well preferred in the compositions of the present invention.

The conditioning compositions of any type may contain active ingredients selected from UV filters, moisturisers, sequestering agents, and natural ingredients.

The moisturizing agents are selected from panthenol, polyols, such as glycerol, polyethylene glycols with molecular weight 200 to 20,000. The moisturizing ingredients can be included in the conditioner compositions at a concentration range of 0.01 - 2.5% by weight calculated to the total composition.

The sequestering agents are selected from polycarboxy acids. The preferred one is ethylene diamine tetraacetic acid, EDTA. Typical useful concentration range for sequestering agents is of 0.01 - 2.5% by weight calculated to the total composition.

Natural plant extracts may be incorporated usually in an amount of about 0.01 % to about 10 %, preferably 0.05 % to 7.5 %, in particular 0.1 % to 5 % by weight, calculated as dry residue thereof to the total composition. Suitable aqueous (e.g. steam-distilled) alcoholic or hydro-alcoholic plant extracts known per se are in particular extracts from leaves, fruits, blossoms, roots, rinds or stems of aloe, pineapple, artichoke, arnica, avocado, valerian, bamboo, henbane, birch, stinging nettle, echinacea, ivy, wild angelica, gentian, ferns, pine needles, silver weed, ginseng, broom, oat, rose hip, hamamelis, hay flowers, elderberry, hop, coltsfoot, currants, chamomile, carrots, chestnuts, clover, burr root, cocoanut, cornflower, lime blossom, lily of the valley, marine algae, balm, mistletoe, passion flower, ratanhia, marigold, rosemary, horse chestnut, pink hawthorn, sage, horsetail, yarrow, primrose, nettle, thyme, walnut, wine leaves, white hawthorn, etc. Suitable trade products are, for example, the various "Extrapon®" products, "Herbasol^{R}", "Sedaplant^{R}" and "Hexaplant^{R}". Extracts and the preparation thereof are also described in "Hagers Handbuch der pharmazeutischen Praxis", 4^{th} Ed..

The viscosity of the conditioning shampoo compositions according to the invention is in the range of 500 and about 20,000 mPa.s at 20°C, preferably 1,000 to 10,000, in particular 1,500 to 8,000 mPa.s at 20°C, measured with Höppler viscosimeter.

Viscosity of shampoo compositions can be adjusted with known viscosity enhancers. The preferred ones are PEG-55 propyleneglycol oleate and PEG-18 glyceryl oleate/cocoate known with the trade names Antil^{R} 141 and 171, respectively and PEG-160 sorbitan triisostearate known with a trade name Rheodol^{R}. It should be noted that in the case that a composition are delivered in the form of a foam from a pump-foamer and/or aerosol can, those compositions should not be thickened and have a viscosity value not more than 500 mPa.s, more preferably 250 mPa.s measured as mentioned above at room temperature.

Viscosity of the non-cleansing conditioning composition may not be more than 50,000 mPa.s at 20°C measured with Brookfield Rheometer at a shear rate of 10 sec⁻¹.

It should especially be noted that the protective effects of the inventive compositions become more and more visible after repeated usage. Especially shine enhancing and prevention of loss of combability are very much pronounced after repeated usage.

The following examples are to illustrate the invention, but not to limit. The compositions according to the invention are prepared by mixing the individual components in water, whereby it is also possible to use pre-mixtures of various ingredients.

### Determination of UV filter deposition onto hair

Beached buffalo hair tresses (weighing approximately 2g) were soaked into preparations (100 ml) for 5 min, 30min 2 h and 24 h and afterwards the swatches were rinsed of with tap water and were dried with an hair dryer. The hair swatch were than soaked into ethanol (96%) for the extraction of UV filter deposited. The same was carried out with a blanc, untreated hair tress. The concentration of Benzophenone-4 is than determined spectrophotometrically by measuring absorbance values of the sample solutions at 288 nm and calculating the concentration of UV filter using a calibration curve made in the same solution, extract of hair. The results are expressed as mg UV filter / g hair.

### Example 1

The following solutions were tested for demonstrating the effect of pH and organic solvent on deposition of anionic water soluble UV filter Benzophenone-4 onto hair.

**Concentration % by weight**

| | Solution A | Solution B |
|---|---|---|
| Benzophenone-4 | 0.3 | 0.3 |
| Cetrimoniumchloride | 0.5 | 0.5 |
| Malic acid | - | 1.0 |
| Lactic acid | - | 0.5 |
| Benzylalcohol | - | 2.5 |
| Ethanol | - | 5.0 |
| Water | to 100 | to 100 |
| pH | 5.5 | 2.5 |

**Table I: The results of UV filter deposition**

| | UV filter deposition mg/g hair | |
|---|---|---|
| Processing time | Solution A | Solution C |
| 5 min | 2.5 | 7.0 |
| 30 min | 3.0 | 14.1 |
| 2 h | 6.5 | 26.2 |
| 24 h | 8.4 | 50.5 |

From the above results it is obvious that by reducing pH of the same composition with addition of malic and lactic acids, the deposition of UV filter enhanced dramatically. Deposition is even further enhanced by addition of organic solvents.

Similar results are observed with the composition below.

### Example 2

**Shampoo composition**

| | |
|---|---|
| Sodium lauryl ether sulfate | 11.0 (% by wt.) |
| Coco glucoside | 4.0 |
| Cocoamidopropyl betaine | 1.5 |
| Cationic polymer (Polyquaternium-10) | 0.2 |
| Benzylalcohol | 1.0 |
| Perfume, preservative | q.s |
| PEG-60-hydrogenated castor oil | 0.5 |
| PEG-18 Glyceryl cocoate/oleate | 2.0 |
| Benzophenone-4 | 0.5 |
| Malic acid | 1.0 |
| Water | ad100.0 |

The pH of the composition is 3.3.

The above shampoo composition was tested with consumers (n=20) aging 20 to 45 years, having ear to shoulder length hair. For the comparative purposes the same composition was prepared and the pH of the composition was adjusted to 5.5 by using sodium hydroxide and benzyl alcohol is excluded and tested again with the 20 volunteers as above (different volunteers were participated the test). After usage period of 3 weeks the test persons were asked to evaluate softness, shine and combability of the hair in a scale of 1 to 5, 1 being the worst and 5 being the best. The results presented below in Table II are the average of the 20 users.

**Table II: Results of the comparative use test.**

| | Example 2 | Example 2 at pH 5.5 (comparative) |
|---|---|---|
| Softness | 4.3 | 2.3 |
| Shine | 4.6 | 1.5 |
| Combability | 4.4 | 2.1 |

From the above results it is obvious that the inventive composition is much more effective than the comparative one.

### Example 3

**Shampoo Composition**

| | |
|---|---|
| Sodium lauryl ether carboxylate (10EO) | 5.0 (% by wt.) |
| Coco glucoside | 5.0 |
| Cocoamidopropyl betaine | 5.0 |
| Cationic polymer (Polyquaternium-7) | 0.2 |
| Benzylalcohol | 1.5 |
| Perfume, preservative | q.s. |
| PEG-60-hydrogenated castor oil | 0.5 |
| PEG-18 Glyceryl cocoate/oleate | 1.0 |
| Benzophenone-4 | 0.5 |
| Lactic acid | 0.40 |
| Malic acid | 0.50 |
| Water | ad 100.0 |

The pH of the composition is 3.5.

### Example 4

**Foam Shampoo**

| | |
|---|---|
| Coco glucoside | 5.0 |
| Cocoamidopropyl betaine | 6.0 |
| Laureth-16 | 4.0 |
| Cationic polymer (Polyquaternium-11) | 0.5 |
| Benzylalcohol | 1.5 |
| Perfume, preservative | q.s. |
| Benzophenone-4 | 0.40 |
| Malic acid | 0.75 |
| Lactic acid | 0.25 |
| Water | ad 100.0 |

The pH of the composition is 3.4. The above composition is a very low viscosity composition, in any case a viscosity lower than 500 mPa.s measured at ambient temperature and with Höppler viscosimeter, confectioned into a pump-foamer as purchased from the company Air-Spray - Germany.

Similarly and aerosol foam shampoo was prepared by confectioning the above composition at a weight ratio of 90/10 ― composition/propellant- using propane-butane mixture as a propellant.

### Example 5

**Hair treatment composition**

| | |
|---|---|
| Cetylstearylalcohol | 5.0 (% by weight) |
| Stearyltrimethylammoniumchlorid | 2.0 |
| Benzylalcohol | 2.5 |
| Benzophenone-4 | 0.3 |
| Benzylalcohol | 4.0 |
| Fragrance, preservative | q.s. |
| Malic acid | 1.0 |
| Wasser | ad 100.0 |

The pH of the composition is 3.0.

### Example 6

**Foam conditioner**

| | |
|---|---|
| Quaternium-80 | 0.2 (Gew.-%) |
| Polyquaternium-11 | 0.7 |
| PEG-160-hydrogenated ricinus oil | 0,5 |
| Fragrance, preservative | q.s. |
| Benzyalcohol | 4.0 |
| Benzophenone-4 | 0.1 |
| Malic acid | 0.5 |
| Lactic acid | 0.2 |
| Wasser | ad 100.0 |

pH of the composition is adjusted to 3.4. The composition is suitable for leave-in and rinse off. In leave-in application, amount used is obviously less than in the case of a rinse of application. The composition is packed into an aerosol can with 90/10 ratio, by weight, liquid composition to propellant. As propellant propane, butane mixture is used.

### Example 7

**Conditioner**

| | |
|---|---|
| Cetylstearylalcohol | 5.0 (% by weight) |
| Cetrimoniumchloride | 1.0 |
| Panthenol | 0,4 |
| Dimethicone | 0.75 |
| Hydroxypropyl Guar Hydroxypropyltrimonium | |
| Chloride | 1.0 |
| Benzylalcohol | 5.0 |
| Benzophenone-4 | 0.3 |
| Avocado extract | 0.5 |
| Fragrance, preservative | q.s. |
| Malic acid | 0.8 |
| Wasser | ad 100.0 |

The pH of the composition is 3.6.

### Example 8

**Conditioner**

| | |
|---|---|
| Cetylstearylalcohol | 5.0 (% by weight) |
| Dioleoylethyldimethylammonium ethosulfate | 1.0 |
| Ceteareth 20 | 1.0 |
| Panthenol | 0,4 |
| Dimethicone | 0.75 |
| Hydroxypropyl Guar Hydroxypropyltrimonium | |
| Chloride | 1.0 |
| Benzophenone-4 | 0.3 |
| Benzylalcohol | 4.0 |
| Avocado extract | 0.5 |
| Fragrance, preservative | q.s. |
| Malic acid | 0.8 |
| Wasser | ad 100.0 |

The pH of the composition is 3.6.

## Claims

1. Conditioning composition for hair **characterized in that**, it comprises at least one UV Filter, at least one hair conditioning agent, at least one organic solvent and at least one acidic component at a concentration of 0.5 to 5% by weight, calculated to total composition, and has a pH less than 4.5, whereby
the UV filter is anionic and water soluble;
the composition comprises at least one hydroxycarboxylic and/or dicarboxylic acid as acidic component;
the composition comprises malic acid as hydroxycarboxylic and/or dicarboxylic acid;
and
the composition comprises organic solvents at a concentration of less than 10% by weight calculated to total concentration.

2. Composition according to claim 1 **characterized in that** the UV filter is present at a concentration of 0.01 to 5% by weight, calculated to total composition.

3. Composition according to claims 1 and 2 **characterized in that** it comprises cationic polymers and/or cationic surfactants as conditioning agent.

4. Composition according to any of the preceding claims **characterized in that** it has a pH in the range of 2.0 to 4.0.

5. Composition according to claim 1 **characterized in that** it comprises lactic acid as hydroxycarboxylic and/or dicarboxylic acid.

6. Composition according to claim 5 **characterized in that** it comprises hydroxycarboxylic and/or dicarboxylic acid at concentration of 0.5 to 5% by weight with the condition that it comprises malic acid at a concentration of not less than 0.5% by weight calculated to total composition.

7. Composition according to claim 6 **characterized in that** it comprises only malic acid as a hydroxycarboxylic acid and/or dicarboxylic acid.

8. Composition according to any of the preceding claims **characterized in that** composition is a cleansing and conditioning composition and comprising at least one surfactant selected from anionic, nonionic and amphoteric or zwitterionic surfactants at a concentration of 1 to 50% by weight calculated to the total composition.

9. Composition according any of the preceding claims **characterized in that** it comprises at least one anionic surfactant and at least one non-ionic surfactant.

10. Composition according to claim 9 **characterized in that** it comprises additionally at least one amphoteric surfactant.

11. Composition according to any of the preceding claims **characterized in that** it is an emulsion and comprises at least one fatty alcohol and an emulsifying surfactant.

12. Use of composition according to any of the preceding claims for depositing at least one UV filter onto hair.

13. Use of composition according claim 12wherein at least one UV filter is selected from anionic, water soluble ones.

## Patentansprüche

1. Konditionierzusammensetzung für Haar, **dadurch gekennzeichnet, dass** sie mindestens einen UV-Filter, mindestens ein Konditionierungsmittel, mindestens ein organisches Lösungsmittel und mindestens eine saure Komponente in einer Konzentration von 0,5 bis 5 Gewichts-%, bezogen auf die Gesamtzusammensetzung, enthält und einen pH-Wert von weniger als 4,5 aufweist, wobei
der UV-Filter anionisch und wasserlöslich ist;
die Zusammensetzung mindestens eine Hydroxycarbonsäure und/oder Dicarbonsäure als saure Komponente enthält;
die Zusammensetzung Äpfelsäure als Hydroxycarbonsäure und/oder Dicarbonsäure enthält;
und
die Zusammensetzung organische Lösungsmittel in einer Konzentration von weniger als 10 Gewichts-% enthält, bezogen auf die Gesamtkonzentration.

2. Zusammensetzung nach Anspruch 1, **dadurch gekennzeichnet, dass** der UV-Filter in einer Konzentration von 0,01 bis 5 Gewichts-% vorliegt, bezogen auf die Gesamtzusammensetzung.

3. Zusammensetzung nach Anspruch 1 und 2, **dadurch gekennzeichnet, dass** sie kationische Polymere und/oder kationische Tenside als Konditionierungsmittel enthält.

4. Zusammensetzung nach einem der vorhergehenden Ansprüche, **dadurch gekennzeichnet, dass** sie einen pH-Wert im Bereich von 2,0 bis 4,0 aufweist.

5. Zusammensetzung nach Anspruch 1, **dadurch gekennzeichnet, dass** sie Milchsäure als Hydroxycarbonsäure und/oder Dicarbonsäure enthält.

6. Zusammensetzung nach Anspruch 5, **dadurch gekennzeichnet, dass** sie Hydroxycarbonsäure und/oder Dicarbonsäure in einer Konzentration von 0,5 bis 5 Gewichts-% enthält, mit der Bedingung, dass sie Äpfelsäure in einer Konzentration von nicht weniger als 0,5 Gewichts-% enthält, bezogen auf die Gesamtzusammensetzung.

7. Zusammensetzung nach Anspruch 6, **dadurch gekennzeichnet, dass** sie nur Äpfelsäure als Hydroxycarbonsäure und/oder Dicarbonsäure enthält.

8. Zusammensetzung nach einem der vorhergehenden Ansprüche, **dadurch gekennzeichnet, dass** die Zusammensetzung eine Reinigungs- und Konditionierzusammensetzung ist und mindestens ein Tensid, ausgewählt aus anionischen, nichtionischen und amphoteren oder zwitterionischen Tensiden, in einer Konzentration von 1 bis 50 Gewichts-% enthält, bezogen auf die Gesamtzusammensetzung.

9. Zusammensetzung nach einem der vorhergehenden Ansprüche, **dadurch gekennzeichnet, dass** sie mindestens ein anionisches Tensid und mindestens ein nichtionisches Tensid enthält.

10. Zusammensetzung nach Anspruch 9, **dadurch gekennzeichnet, dass** sie zusätzlich mindestens ein amphoteres Tensid enthält.

11. Zusammensetzung nach einem der vorhergehenden Ansprüche, **dadurch gekennzeichnet, dass** sie eine Emulsion ist und mindestens einen Fettalkohol und ein emulgierendes Tensid enthält.

12. Verwendung einer Zusammensetzung nach einem der vorhergehenden Ansprüche zum Aufbringen von mindestens einem UV-Filter auf Haar.

13. Verwendung einer Zusammensetzung nach Anspruch 12, wobei mindestens ein UV-Filter aus anionischen wasserlöslichen ausgewählt ist.

## Revendications

1. Composition de conditionnement pour les cheveux, **caractérisée en ce qu'**elle comprend au moins un filtre UV, au moins un agent de conditionnement capillaire, au moins un solvant organique et au moins un composant acide à une concentration de 0,5 à 5 % en poids, calculée par rapport à la composition totale, et a un pH inférieur à 4,5
le filtre UV étant anionique et soluble dans l'eau ;
la composition comprenant au moins un acide hydroxycarboxylique et/ou un acide dicarboxylique en tant que composant acide ;
la composition comprenant de l'acide malique en tant qu'acide hydroxycarboxylique et/ou acide dicarboxylique ;
et
la composition comprenant des solvants organiques à une concentration inférieure à 10 % en poids calculée par rapport à la composition totale.

2. Composition selon la revendication 1, **caractérisée en ce que** le filtre UV est présent à une concentration de 0,01 à 5 % en poids, calculée par rapport à la composition totale.

3. Composition selon les revendications 1 et 2, **caractérisée en ce qu'**elle comprend des polymères cationiques et/ou des tensioactifs cationiques en tant qu'agents de conditionnement.

4. Composition selon l'une quelconque des revendications précédentes, **caractérisée en ce qu'**elle a un pH dans la gamme de 0,2 à 4,0.

5. Composition selon la revendication 1, **caractérisée en ce qu'**elle comprend de l'acide lactique en tant qu'acide hydroxycarboxylique et/ou acide dicarboxylique.

6. Composition selon la revendication 5, **caractérisée en ce qu'**elle comprend de l'acide hydroxycarboxylique et/ou de l'acide dicarboxylique à une concentration de 0,5 à 5 % en poids avec la condition qu'elle comprenne de l'acide malique à une concentration qui ne doit pas être inférieure à 0,5 % en poids calculée par rapport à la composition totale.

7. Composition selon la revendication 6, **caractérisée en ce qu'**elle comprend uniquement de l'acide malique en tant qu'acide hydroxycarboxylique et/ou acide dicarboxylique.

8. Composition selon l'une quelconque des revendications précédentes, **caractérisée en ce que** la composition est une composition de nettoyage et de conditionnement et comprend au moins un tensioactif, choisi parmi les tensioactifs anioniques, non ioniques et amphotères ou zwitterioniques, à une concentration de 1 à 50 % en poids calculée par rapport à la composition totale.

9. Composition selon l'une quelconque des revendications précédentes, **caractérisée en ce qu'**elle comprend au moins un tensioactif anionique et au moins un tensioactif non ionique.

10. Composition selon la revendication 9, **caractérisée en ce qu'**elle comprend de manière additionnelle au moins un tensioactif amphotère.

11. Composition selon l'une quelconque des revendications précédentes, **caractérisée en ce qu'**elle est une émulsion et comprend au moins un alcool gras et un tensioactif émulsionnant.

12. Utilisation de la composition selon l'une quelconque des revendications précédentes pour déposer au moins un filtre UV sur les cheveux.

13. Utilisation de la composition selon la revendication 12, où au moins un filtre UV est choisi parmi ceux anioniques, solubles dans l'eau.
